# EUROPEAN PATENT APPLICATION

(11) **EP 0 955 065 A1**
(43) Date of publication of application: **10.11.1999**
(21) Application number: 99200448.1
(22) Date of filing: 16.02.1999
(51) Int. Cl.: A61K 49/00

(54) **Polyethylene glycol (PEG 4000) in a contrast medium composition for echography**

(30) Priority: 18.02.1998 IT MI980303
(71) Applicant: Promefarm s.r.l., 20129 Milano (IT)
(72) Inventor: Corazziari, Enrico, 00168 Roma (IT)
(74) Representative: Marchi, Massimo, Dr.

(57) **Abstract**

Use of a polyethylene glycol compound and an agent capable of reducing the amount of intestinal gases in the preparation of a composition useful as a contrast medium in echography.

## Description

The present invention relates to the use of a polyethylene glycol compound in the preparation of a composition useful as a contrast medium in echography of the gastrointestinal apparatus, and to a composition comprising a polyethylene glycol compound (PEG), an agent capable of reducing the amount of intestinal gases and a prokinetic agent.

It is known that the diagnostic tests of the gastrointestinal apparatus are generally performed by endoscopy and/or radiology.

Owing to the inherent properties of the technique, however, the endoscopy diagnostic capabilities are limited to the proximal and distal tract of the digestive apparatus, that is esophagus, stomach, duodenum and colon.

This technique cannot be therefore used in diagnostic tests of the mesenteric tenuis.

Furthermore, endoscopy is an invasive technique with the risk of complications and, therefore, cannot be used as a screening test.

Radiology, on the other hand, although being capable of evaluating the morphology of the whole gastrointestinal tract, has the following drawbacks:
- it allows an indirect evaluation only of the inner surface of the intestinal wall;
- it exposes the patient to the risks connected to radiation and, therefore, cannot be used in pregnancy, has to be used with care in children, cannot be repeated in the same patient as it would be required, for example, in the follow up of patients suffering from intestinal chronic diseases, and is not suitable as a screening methodology in gastrointestinal troubles;
- it needs rooms protected from radiation and this bears on the costs; and
- it requires an invasive approach for the study of the mesenteric tenuis by enema.

Therefore the need is still much felt of a technique allowing to explore the whole intestinal wall, easy to be performed, not invasive, not exposing to radiation risks and which can be used in children and during the pregnancy.

The technique which more satisfies the aforesaid requirements is echography.

Indeed it is not invasive, it is of easy execution, can be repeated and is not expensive as it does not imply special wards and specialized technical staff, and can be carried out without special preparations in all the patients and in out-patients' department.

So far, however, echography was never used to visualize the wall of the whole gastrointestinal tract. This is due to the lack of a composition capable of relaxing the intestinal loops for a sufficiently long period and which is, in the meanwhile, capable of causing a good reflection of the high-frequency ultrasound waves sent towards the intestinal tract, thus giving a satisfactory intensity and contrast to the produced image.

However, the morphology and the thinness (2-4 mm) of the gastrointestinal tract walls make them difficult to be separated one from the other and scantily differentiable from other loops and other adjacent structures.

Furthermore, the presence of intestinal gases, widely and randomly spaced in the gastrointestinal tract, is responsible for the lack of a real lumen and this is a further obstacle to test the whole gastrointestinal tract in a complete and sequential way.

The above obstacles could be overcome only if an anechogen medium exists capable of relaxing the gastrointestinal tract and separating one from the other the walls of each tract.

Now it has been found that the above requirements are met by using a polyethylene glycol compound and an agent capable of reducing the amount of intestinal gases.

Therefore, it is a first object of this invention to provide the use of a polyethylene glycol compound and an agent capable of reducing the intestinal gas amount in the preparation of a composition useful as a contrast medium in echography.

Preferably, the polyethylene glycol compound is PEG 4000.

Preferably the agent capable of reducing intestinal gases is selected from the group comprising simethicone (activated dimethicone) and activated carbon.

Preferably the composition of this invention further comprises at least one agent capable of making isoosmotic the aqueous solution obtained when dissolving said composition in water.

Typical examples of said agent are the mineral salts such as sodium sulfate, sodium chloride, sodium bicarbonate, potassium chloride and magnesium sulfate and mixtures thereof.

The above composition may further comprise flavours, sweeteners and other conventional ingredients.

Typically said composition for a diagnostic test in an adult patient shall comprise from 10 to 40 g, preferably from 15 to 30 g, still more preferably from 15 to 20 g of a polyethylene glycol compound .

When the agent used to reduce the amount of intestinal gases is simethicone, the dose thereof shall be typically comprised from 50 to 400 mg, preferably from 100 to 250 mg, still more preferably from 115 to 230 mg.

According to a preferred embodiment, the above composition is administered concomitantly with a prokinetic agent.

Preferably said prokinetic agent is selected from the group comprising domperidone and cisapride, and still more preferably is the domperidone.

In the case of domperidone, a typical dosage is of from 5 to 30 mg, and is administered in a single dose.

In the case of cisapride, a typical dosage is of from 5 to 20 mg, and is administered in a single dose.

The prokinetic agent may be also incorporated into the above mentioned composition. This composition is novel.

Therefore, it is a second object of this invention to provide a composition, characterized in that it comprises a polyethylene glycol compound, an agent capable of reducing the amount of intestinal gases and a prokinetic agent.

Preferably said prokinetic agent is selected from the group comprising domperidone and cisapride.

Preferably, the polyethylene glycol compound is PEG 4000, and the agent capable of reducing the amount of intestinal gases is selected from the group comprising simethicone (activated dimethicone) and activated carbon.

Preferably the composition of this invention further comprises at least one agent capable of making isoosmotic an aqueous solution obtained when dissolving said composition in water.

When the prokinetic agent is domperidone or cisapride, their preferred dosage in the composition of this invention shall be as already indicated above.

The dosage forms of the composition of this invention are preferably aqueous solutions or solid forms, such as powders, pellets or pills suitable to form extemporaneous aqueous solutions.

Typically, for the echographic diagnostic test in an adult patient, a solid presentation form according to this invention is dissolved in a amount of water of from 100 to 800 ml. Preferably, the amount of water is of from 120 to 300 ml.

The patient is then submitted to the echographic test after a time of from few minutes to 30 minutes, preferably from 5 to 15 minutes after administration of said aqueous solution.

The time useful for the carrying out of the echographic test is of about 50 minutes from the administration of said aqueous solution.

In children, the dose shall depend on the age and can be easily determined by a person skilled in the art in view of the dosages mentioned above in relation to the adult.

Peculiar features of a composition of this invention are that it is anechogen and capable of displaying and identifying each tract of the gastrointestinal hank as separated from the other. Indeed the anechogenity of the medium makes very clear, for contrast, and therefore analyzable, the multilayer structure of the thin gastrointestinal wall of each tract. Furthermore during its progression, the medium makes visible in an uniform and uninterrupted way all the tracts of the intestinal loops. It is thus possible to diagnose the presence of any pathologic alterations, such as tumours and inflammations of the intestinal walls, such as for example Crohn's disease, lymphomas, polyps, adenocarcinomas and appendix alterations.

More particularly, the composition of this invention allows:
a) an echographic visualization of the whole intestinal tract, duodenum to cecum;
b) a precise evaluation of the condition of the intestinal loops wall;
c) a non-invasive diagnosis of pathologic alterations interesting the small intestine;
d) the production of an echographic image as reliable and trustworthy as the radiographic one.

Furthermore the composition of this invention makes the echographic method sensible and specific in displaying the layer of the wall of all the small intestine. Hence, the easiness of execution of the echographic method joined to its non-invasive nature, makes it suitable for the:
1. screening of patients suffering from gastrointestinal troubles;
2. follow up of patients suffering from chronic inflammatory intestinal diseases;
3. study of the intestinal tract whenever there are symptoms even in transitory pathologic conditions and in acute conditions.

The following examples are intended to illustrate this invention without, however, limiting it in any way.

### COMPARATIVE EXAMPLE 1

As a comparison composition it has been used a commercial isoosmotic composition SELG™ 1000 (sold by Promefarm) conventionally used for intestinal washings before performing colonscopy or double-contrast barium enema.

An envelope of SELG™ 1000 contained 70 g of a powder to be dissolved in water to form 1 litre of an aqueous solution, and the said powder had the following composition:

| ingredient | g/l | % |
|---|---|---|
| PEG 4000 | 58.32 | 88.31 |
| anhydrous sodium sulfate | 5.69 | 8.13 |
| sodium bicarbonate | 1.69 | 2.14 |
| sodium chloride | 1.46 | 2.08 |
| potassium chloride | 0.74 | 1.06 |
| sweeteners and flavours | q.s. | 3.01 |

The above comparison aqueous solution was orally administered to 8 healthy people (4 females and 4 males having an age comprised between 29 and 46 years) on an empty stomach in increasing doses 300 ml to 820 ml, 10 minutes before the echographic test.

At the dose of 500 ml, after 15-20 minutes from the test beginning, in all the people it was possible to visualize, by abdominal echography, the duodenum, the mesenteric tenuis and the cecum, but the quality of the image was not satisfying.

### EXAMPLE 1

It was carried out in the same way as the preceding Comparative Example 1, except for the use of a composition according to this invention.

The formulation of the composition to be dissolved in 1 litre of water was the following:

| ingredient | g/l | % |
|---|---|---|
| PEG 4000 | 105.00 | 94.63 |
| sodium bicarbonate | 1.43 | 1.28 |
| sodium chloride | 2.80 | 2.523 |
| potassium chloride | 0.36 | 0.324 |
| simethicone | 0.900 | 0.27 |
| acesulphame | 0.16 | 0.144 |
| sweeteners and flavours | 0.90 | 0.81 |

In all the people it was possible to visualize by abdominal echography the duodenum, the mesenteric tenuis and the cecum. More particularly it was possible to visualize, following the whole way, all the intestinal loops from duodenum to ileum up to the last intestinal loop and it was possible to evaluate:
- the diameter of the intestinal lumen which, measured in tracts without contractions, was not higher than 2 cm;
- the thickness of the intestinal wall resulted to be comprised between 1 and 3 mm; and
- the multilayer structure of the intestinal wall.

With respect to the Comparative Example 1, the addition of simethicone has given an echographic image by far better and the greater PEG concentration has allowed to reduce in a considerable way the amount of solution to be administered for performing the echographic test.

### EXAMPLE 2

5 of the people of the previous Comparative Example 1 were submitted to a second experiment addressed to find which is the minimum dose of the solution of Example 1 that allows to visualize duodenum, tenuis and cecum.

The minimum dose, ingested all at once, which has allowed to echographically observe the whole blind and small intestine varied, from people to people, within the interval 130 ml to 250 ml (minimum weighed average dose = 200 ml).

### EXAMPLE 3

On 4 healthy volunteers (3 females and 1 male having an age comprised between 25 and 46 years) it was carried out a double blind experiment by administering on an empty stomach 300 ml of the solution of the previous Example 1, a first time without and a second time concomitantly with 20 mg of domperidone.

Each volunteer was examined in two different days randomly.

The results obtained have shown that:
- in the people who received the composition without domperidone, the visualization time of the last intestinal loop and the cecum was, on the average, of 16 minutes from the end of the administration; while
- in the people who received the composition concomitantly with domperidone, the visualization time of the last intestinal loop and the cecum was, on the average, of 8 minutes from the end of the administration;
- the image quality, however, did not change.

### EXAMPLE 4

The solution of the preceding Example 1 (500 ml) was orally administered to 26 patients (14 females and 12 males having an age comprised between 20 and 60 years) on an empty stomach suffering from diarrhoea and abdominal pain consistent with the diagnostic hypothesis of an alteration of the small intestine. The standard reference examination was represented by the radiologic examination of the digestive apparatus with seriated study of tenuis. The echographic evaluation was carried out independently of the radiologic one by an echographic operator who did not know the radiologic result.

The results were the following:
- in 14 patients the echographic examination did not show any alterations and the radiologic examination confirmed the lack of alterations of the intestinal wall and lumen;
- in 12 patients the echographic examination showed the presence of alterations of the intestinal wall and/or lumen, and the radiological examination confirmed the existence of alterations in all the cases except one.

Therefore the composition of this invention makes the echographic image reliable and trustworthy as the radiographic one.

## Claims

1. Use of a polyethylene glycol compound and an agent capable of reducing the amount of intestinal gases in the preparation of a composition useful as a contrast medium in echography.

2. The use of claim 1, characterized in that the polyethylene glycol compound is PEG 4000.

3. The use of claim 1 or 2, characterized in that the agent capable of reducing the amount of intestinal gases is simethicone (activated dimethicone) or activated carbon.

4. The use of any of the preceding claims 1 to 3, characterized in that said composition further comprises at least one agent capable of making isoosmotic the aqueous solution obtained when dissolving the composition in water.

5. A composition useful as a contrast medium in echography,
characterized in that it comprises a polyethylene glycol compound, an agent capable of reducing the amount of intestinal gases and a prokinetic agent.

6. The composition of claim 5, characterized in that the prokinetic agent is domperidone or cisapride.

7. The composition of claim 5 or 6, characterized in that the polyethylene glycol compound is PEG 4000.

8. The composition of any of claims 5 to 7, characterized in that the agent capable of reducing the amount of intestinal gases is simethicone (activated dimethicone) or activated carbon.

9. The composition of any of claims 5 to 8, characterized in that it further comprises at least one agent capable of making isoosmotic the aqueous solution obtained when dissolving the composition in water.
